(19) Europäisches Patentamt

European Patent Office

Office européen des brevets

(11) **EP 0 856 304 B1**

(12) **EUROPÄISCHE PATENTSCHRIFT**

(45) Veröffentlichungstag und Bekanntmachung des Hinweises auf die Patenterteilung:
**12.06.2002 Patentblatt 2002/24**

(51) Int Cl.⁷: **A61K 7/06**

(21) Anmeldenummer: **97117646.6**

(22) Anmeldetag: **11.10.1997**

(54) **Natürliche Öle enthaltende Mittel zur Festigung von Haaren**

Composition containing natural oils for fixing the hair

Composition contenant des huiles naturelles pour la fixation de la coiffure

(84) Benannte Vertragsstaaten:
**DE ES FR GB IT**

(30) Priorität: **15.03.1997 DE 19710874**
**22.01.1997 DE 19702156**

(43) Veröffentlichungstag der Anmeldung:
**05.08.1998 Patentblatt 1998/32**

(73) Patentinhaber: **Wella Aktiengesellschaft**
**64274 Darmstadt (DE)**

(72) Erfinder:
• **Schmenger, Jürgen**
**64331 Weiterstadt (DE)**
• **Karlen, Thomas, Dr.**
**CH-3013 Bern (CH)**
• **Kripp, Thomas, Dr.**
**64407 Fränkisch-Crumbach (DE)**
• **Titze, Hans-Jürgen**
**64401 Gross-Bieberau (DE)**
• **Steinbrecht, Karin, Dr.**
**64372 Ober-Ramstadt (DE)**
• **Birkel, Susanne, Dr.**
**64380 Rossdorf (DE)**
• **Lede, Michael**
**63225 Langen (DE)**
• **Borth, Silvia**
**64407 Fränkisch-Crumbach (DE)**

(56) Entgegenhaltungen:
**WO-A-95/31176**

**Beschreibung**

Gegenstand der vorliegenden Erfindung ist ein Mittel zur Behandlung von Haaren, das zur Herstellung einer Frisur und zur Fixierung der Haare vorteilhaft eingesetzt werden kann.

Es sind bereits zahlreiche Produkte bekannt, welche den Haaren durch Polymerzusatz Halt, Volumen, Elastizität, Sprungkraft und Glanz verleihen. Diese als Stylingprodukte bezeichneten Präparate erleichtern als Gel die Formgebung, verbessern als Haarspray den Stand und als Festigerschaum das Volumen des Haares. Eine starre, steife Festigung ist oftmals der Nachteil bekannter Stylingprodukte. Stylingprodukte mit natürlicher Spungkraft und erhöhter Elastizität lassen Haare natürlicher erscheinen.

Es stellt sich deshalb die Aufgabe, bei vorhandenen Stylingprodukten durch geeignete Zusätze die Elastizität, die Sprungkraft und den Glanz des menschlichen Haares weiter zu verbessern. Diese Aufgabe läßt sich erfindungsgemäß besonders eindrucksvoll durch die Zugabe von bestimmten natürlichen Ölen zu den Rezepturen bekannter Stylingprodukte lösen.

Es wurde nun gefunden, daß ein Haarbehandlungsmittel die vorteilhaften Eigenschaften der bisher bekannten Stylingprodukte noch übertrifft, wenn es eine Kombination von

    (A) mindestens einem Öl ausgewählt aus Zedemholzöl, Krambeöl, Leindotteröl und Hanföl und
    (B) mindestens einem filmbildenden, haarfestigenden Polymer,
    und gegebenenfalls weitere, für Haarkosmetika übliche Zusatzstoffe enthält.

Durch Zusatz der natürlichen Öle werden die Elastizität, die Sprungkraft und der Haarglanz erheblich verbessert. Führt man eine Haarbehandlung mit Stylingprodukten durch, die mit oder ohne Zugabe der natürlichen Öle hergestellt waren, zeigen die unter Verwendung der mindestens eines der genannten natürlichen Öle enthaltenden Präparate behandelten Haare bei einer sensorischen Beurteilung ein elastischeres Anfühlen. Anwendungstechnische Versuche beim Halbseitenvergleich zeigen, daß die Zugabe von Zedemholzöl, Krambeöl und/oder Leindotteröl zum Stylingprodukt den Glanz der damit behandelten Haare erhöht. Es war überraschend, daß gerade durch den Zusatz von Zedemholzöl, Krambeöl und/oder Leindotteröl zu herkömmlichen Stylingprodukten eine bemerkenswerte Erhöhung des Haarglanzes erreicht werden kann. Messungen von Wasserdampfaufnahmen zeigen, daß Polymerfilme mit einem Gehalt an Leindotteröl oder Krambeöl eine deutlich erhöhte Resistenz gegenüber Feuchtigkeit aufweisen.

Überraschenderweise wird durch Zusatz von Hanföl die Elastizität und die Sprungkraft des Haares verbessert. Sprungkraft beziehungsweise Elastizität ist ein Maß für die Bewegung einer Haarlocke nach einer äußeren Krafteinwirkung, die physikalisch als eine gedämpfte Schwingung angesehen werden kann. Führt man eine Haarbehandlung mit filmbildende Polymere enthaltenden Stylingprodukten durch, die mit oder ohne Zugabe von Hanföl hergestellt waren, zeigen die unter Verwendung von Hanföl erhaltenen Lösungen bei einer meßtechnischen Untersuchung der Bruchkräfte von Polymeren einen signifikant verringerten Meßwert der Bruchkraft, was gleichbedeutend mit einer Zunahme der Elastizität ist. Eine meßtechnische Untersuchung der Sprungkraft von Polymeren mit und ohne Hanföl bestätigen die Elastizitätszunahme von Polymeren mit Zusatz an Hanföl.

Krambeöl und Leindotteröl werden durch Kaltpressung mit einer Ölpresse aus der Ölpflanze Krambe (Grambe abyssinica) beziehungsweise aus den Samen von Leindotter (Camelina, Öldotter) gewonnen und können ohne weitere Reinigung oder chemische Nachbehandlung eingesetzt werden. Die Öle zeichnen sich durch einen hohen Anteil an C16- bis C24-Fettsäuren, insbesondere durch einen hohen Gehalt an ungesättigten Fettsäuren wie Oel-, Linol-, Linolen-, Eicosen-, Eicosadien-, Eruca-, Docosadien- und Nervonsäure aus. So hat Krambeöl einen Erucasäureanteil von 55 bis 65 Prozent und Leindotteröl einen Eicosen- bzw. Eicosadiensäureanteil von 15 bis 25 Prozent.

Zedemholzöl ist eine Sammelbezeichnung für ätherische Öle, die durch Wasserdampfdestillation aus dem Holz verschiedener Zedemarten erhalten werden. Sie enthalten vor allem Sesquiterpene und variieren in ihrer Zusammensetzung bei der Gewinnung aus unterschiedlichen Zedemarten nur wenig. Zedemholzöl ist im Handel erhältlich und kann beispielsweise von der Firma IFF, Hamburg oder in gereinigter Form von der Firma Merck, Darmstadt, erworben werden.

Hanföl ist das aus dem Korn der Hanfpflanze gepreßte Öl und ist kommerziell erhältlich. Hanföl besteht überwiegend aus ungesättigten, essentiellen Fettsäuren. Das Fettsäurespektrum einer typischen Zusammensetzung ist: ca. 57 % zweifach ungesättigte Linolsäure, ca. 18 % dreifach ungesättigte Linolensäure, ca. 12 % einfach ungesättigte Ölsäure, ca. 7 % Palmitinsäure, ca. 3 % Stearinsäure, ca. 2 % Gamma-Linolensäure.

Erfindungsgemäß werden die natürlichen Öle in einer Menge von 0,01 bis 10 Gewichtsprozent, vorzugsweise in einer Menge von 0,05 bis 3,0 Gewichtsprozent zusammen mit einem synthetischen oder natürlichen, nichtionischen, kationischen, anionischen oder amphoteren, filmbildenden Polymeren eingesetzt. Ein derartiges Polymer, das in Mengen von 0,01 bis 50, vorzugsweise in Mengen von 0,05 bis 20 Gewichtsprozent im Haarbehandlungsmittel enthalten ist, kann auch aus einem Gemisch mehrerer Polymerer bestehen und durch den Zusatz weiterer Polymerer mit verdickender Wirkung in seinen haarfestigenden Eigenschaften noch modifiziert werden.

**EP 0 856 304 B1**

Unter filmbildenden, haarfestigenden Polymeren werden erfindungsgemäß solche Polymere verstanden, die bei Anwendung in 0,01 bis 5%iger wäßriger, alkoholischer oder wäßrig-alkoholischer Lösung in der Lage sind, auf dem Haar einen Polymerfilm abzuscheiden und auf diese Weise das Haar zu festigen.

Als geeignete synthetische, nichtionische, filmbildende, haarfestigende Polymere können in dem erfindungsgemäßen Haarbehandlungsmittel Homopolymere des Vinylpyrrolidons, Homopolymere des N-Vinylformamids, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykole mit einem Molekulargewicht von 800 bis 20.000 g/mol eingesetzt werden.

Unter den geeigneten synthetischen, filmbildenden anionischen Polymeren sind beispielsweise Crotonsäure-Vinylacetat-Copolymere und Terpolymere aus Acrylsäure, Ethylacrylat und N-t-Butylacrylamid geeignet.

Natürliche filmbildende Polymere oder daraus durch chemische Umwandlung hergestellte Derivate können in dem erfindungsgemäßen Haarbehandlungsmittel ebenfalls eingesetzt werden. Bewährt haben sich niedermolekulares Chitosan mit einem Molekulargewicht von 30.000 bis 70.000 g/mol oder hochmolekulares Chitosan, Gemische aus Oligo-, Mono- und Disacchariden, chinesisches Balsamharz, Cellulosederivate wie Hydroxypropylcellulose mit einem Molekulargewicht von 30.000 bis 50.000 g/mol, oder Schellack in neutralisierter oder unneutralisierter Form.

Auch amphotere Polymere können in dem erfindungsgemäßen Haarbehandlungsmittel eingesetzt werden. Geeignet sind zum Beispiel Copolymere aus Octylacrylamid, t-Butylaminoethylmethacrylat sowie zwei oder mehr Monomeren aus der Gruppe Acrylsäure, Methacrylsäure und deren einfachen Ester.

Unter den kationischen Polymeren, die erfindungsgemäß eingesetzt werden können, sind Copolymere des Vinylpyrrolidons mit quatemierten Derivaten des Dialkylaminoacrylats- und -methacrylats, wie beispielsweise mit Diethylsulfat quatemierte Vinylpyrrolidon/ Dimethylaminomethacrylat Copolymere zu nennen. Weitere geeignete kationische Polymere sind beispielsweise das Copolymerisat des Vinylpyrrolidons mit Vinylimidazoliummethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcarprolactam, das quaternierte Ammoniumsalz, hergestellt aus Hydroxyethylcellulose und einem mit Trimethylammonium substituierten Epoxid, das Vinylpyrrolidon/Methacrylamidopropyltrimethylammoniumchlorid Copolymer und diquatemäre Polydimethylsiloxane.

Die Konsistenz des erfindungsgemäßen Haarbehandlungsmittels kann durch den Zusatz von Verdickem verbessert werden. Hierfür sind beispielsweise Homopolymere der Acrylsäure mit einem Molekulargewicht von 2.000.000 bis 6.000.000 g/mol geeignet. Auch Copolymere aus Acrylsäure und Acrylamid (Natriumsalz) mit einem Molekulargewicht von 2.000.000 bis 6.000.000 g/mol und Sclerotium Gum sind geeignet. Auch geeignet sind Copolymere der Acrylsäure und der Methacrylsäure.

Das erfindungsgemäße Haarbehandlungsmittel liegt im allgemeinen als wäßrige, alkoholische oder wäßrigalkoholische Lösung vor. Geeignete Lösungsmittel sind beispielsweise aliphatische Alkohole mit 1 bis 4 Kohlenstoffatomen oder ein Gemisch von Wasser mit einem der genannten Alkohole. Es können jedoch auch andere organische Lösungsmittel eingesetzt werden, wobei insbesondere unverzweigte oder verzweigte Kohlenwasserstoffe wie Pentan, Hexan, Isopentan und zyklische Kohlenwasserstoffe wie Cyclopentan und Cyclohexan zu nennen sind. Die Lösungsmittel liegen in einer Menge von 0,5 bis 90 Gewichtsprozent, bevorzugt in einer Menge von 5 bis 50 Gewichtsprozent vor.

Üblicherweise können dem erfindungsgemäßen Haarbehandlungsmittel weitere bekannte kosmetische Zusatzstoffe beigefügt werden, zum Beispiel nichtfestigende, nichtionische Polymere wie Polyethylenglykol mit einem Molekulargewicht von etwa 600 g/mol, nichtfestigende, anionische und natürliche Polymere sowie deren Mischungen in einer Menge von vorzugsweise 0,01 bis 50 Gewichtsprozent. Auch Parfümöle in einer Menge von 0,01 bis 5 Gewichtsprozent, Trübungsmittel wie Ethylenglykoldistearat in einer Menge von 0,01 bis 5 Gewichtsprozent, Netrmittel oder Emulgatoren aus den Klassen der anionischen, kationischen, amphoteren oder nichtionogenen Tenside wie Fettalkoholsulfate, ethoxylierte Fettalkohole, Fettsäurealkanolamide wie die Ester der hydrierten Rizinusölfettsäuren in einer Menge von 0,1 bis 30 Gewichtsprozent, außerdem Feuchthaltemittel, Farbstoffe, Lichtschutzmittel, Antioxidantien, vorzugsweise Vitamin E Acetat (Tocopherol) und Konservierungsstoffe in einer Menge von 0,01 bis 10 Gewichtsprozent.

Das erfindungsgemäße Haarbehandlungsmittel kann weiterhin durch Zusatz von Silikonpolymeren verbessert werden, wie beispielsweise Polydimethylsiloxan (INCI: Dimethicon), $\alpha$-Hydro-$\omega$-hydroxypolyoxydimethylsilylen (INCI: Dimethiconol), cyclisches Dimethylpolysiloxan (INCI: Cyclomethicon), Trimethyl(octadecyloxy)silan (INCI: Stearoxytrimethylsilan), Dimethylsiloxan/Glykol Copolymer (INCI: Dimethicon Copolyol), Dimethylsiloxan/ Aminoalkylsiloxan Copolymer mit Hydroxyendgruppen (INCI: Amodimethicon), Monomethylpolysiloxan mit Laurylseitenketten und Polyoxyethylen- und/oder Polyoxypropylenendketten, (INCI: Laurylmethicon Copolyol), Dimethylsiloxan/Glykol Copolymeracetat (INCI: Dimethiconcopolyol Acetat), Dimethylsiloxan/Aminoalkylsiloxan Copolymer mit Trimethylsilylendgruppen (INCI: Trimethylsilylamodimethicon). Bevorzugte Silikonpolymere sind Dimethicone, Cyclomethicone und Dimethiconole.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet wie zum Beispiel eine Mischung aus Dimethicon und Dimethiconol.

Das erfindungsgemäße Mittel kann in verschiedenen Applikationsformen Anwendung finden, wie beispielsweise in Aerosolzubereitungen als Schaum oder als Spray, desweiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Gel, Wachs oder Mikroemulsion.

Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol- Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur oder zur Pflege der Haare verstanden werden.

Desweiteren ist mit der erfindungsgemäßen Polymerkombination die Herstellung von Konzentraten möglich, welche einen verringerten Wassergehalt und/oder Lösungsmittelgehalt aufweisen. Die Konzentrate werden nach Transport und ggf. Lagerung durch Zugabe der erforderlichen Menge Wasser und/oder Lösungsmittel in ein anwendungsfähiges Haarbehandlungsmittel überführt.

Die nachfolgenden Beispiele sollen den Gegenstand der Erfindung näher erläutern.

| Beispiel 1: Schaumfestiger mit starker Festigung | |
|---|---|
| 0,25 g | Hanföl |
| 0,20 g | 2-Pyrrolidon-5-carbonsäure |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |
| 96,70 g | Wasser |
| 100,00 g | |

| Beispiel 2: Flüssigfestiger mit starker Festigung | |
|---|---|
| 0,20 g | Hanföl |
| 1,00 g | Vinylacetat-Crotonsäure-Copolymer, 60%-ige Lösung in Isopropanol/Wasser (ARISTOFLEX® der Firma Hoechst/Deutschland) |
| 2,58 g | Glycerin |
| 50,00 g | Ethanol |
| 0,50 g | Parfümöl |
| 45,72 g | Wasser |
| 100,00 g | |

| Beispiel 3: Gelförmiger Festiger mit starker Festigung | |
|---|---|
| 0,15 g | Hanföl |
| 0,10 g | 2-Pyrrolidon-5-carbonsäure |
| 3,00 g | Vinylpyrrolidon-Vinylacetat-Copolymer |
| 1,00 g | Hydroxyethylcellulose |
| 5,00 g | Glycerin |
| 0,40 g | hydriertes Rizinusöl, ethoxyliert mit 45 mol Ethylenoxid |
| 90,35 g | Wasser |
| 100,00 g | |

| Beispiel 4: Schaumfestiger für strapaziertes Haar | |
|---|---|
| 0,15 g | Hanföl |
| 2,00 g | Polyvinylpyrrolidon |
| 0,30 g | $\alpha$-Hydro-$\omega$-hydroxy-polyoxydimethylsilylen, 13%ig in Cyclodimethylpolysiloxan (Dow Coming Q2 1401 der Firma Dow Coming Europe/Belgien) |
| 0,40 g | hydriertes Rizinusöl, ethoxyliert mit 45 mol Ethylenoxid |
| 5,00 g | 1,2-Propylenglykol |
| 0,10 g | Cetyltrimethylammoniumchlorid |
| 0,10 g | Isopropanol |
| 91,95 g | Wasser |
| 100,00 g | |

| Beispiel 5: Schaumfestiger für extra starken Halt | |
|---|---|
| 0,15 g | Hanföl |
| 0,40 g | hydriertes Rizinusöl, ethoxyliert mit 45 mol Ethylenoxid |
| 0,10 g | 2-Pyrrolidon-5-carbonsäure |
| 2,00 g | Polyvinylpyrrolidon |
| 2,00 g | Glukosesirup, 64% Oligosaccharide (C-PUR® 01924 der Firma Cerestar/Belgien) |
| 0,30 g | Polyoxyethylen(4)laurylether |
| 0,10 g | Cetyltrimethylammoniumchlorid |
| 0,10 g | Isopropanol |
| 40,00 g | Ethanol |
| 54,85 g | Wasser |
| 100,00 g | |

| Beispiel 6: Gelförmiger Festiger mit natürlichen Polymeren | |
|---|---|
| 0,15 g | Hanföl |
| 0,60 g | hydriertes Rizinusöl, ethoxyliert mit 45 mol Ethylenoxid |
| 5,00 g | Glukosesirup, 64% Oligosaccharide (C-PUR® 01924 der Firma Cerestar/Belgien) |
| 5,00 g | Sorbitsirup |
| 2,00 g | Mutterkomharz (Sclerotium Gum) |
| 87,25 g | Wasser |
| 100,00 g | |

| Beispiel 7: Sprühfestiger zum Fönen | |
|---|---|
| 0,10 g | Hanföl |

(fortgesetzt)

| Beispiel 7: Sprühfestiger zum Fönen | |
|---|---|
| 0,20 g | hydriertes Rizinusöl, ethoxyliert mit 45 mol Ethylenoxid |
| 0,75 g | Vinylpyrrolidon-Vinylacetat-Copolymer |
| 30,00 g | Ethanol |
| 3,00 g | Propylenglykol |
| 0,75 g | Isopropanol |
| 65,20 g | Wasser |
| 100,00 g | |

| Beispiel 8: Non-Aerosol Haarspray | |
|---|---|
| 0,15 g | Hanföl |
| 3,00 g | Vinylpyrrolidon-Vinylacetat-Copolymer |
| 0,10 g | 2-Pyrrolidon-5-carbonsäure |
| 96,75 g | Ethanol |
| 100,00 g | |

| Beispiel 9: Farbfestiger | |
|---|---|
| 0,2500 g | Hanföl |
| 0,6000 g | hydriertes Rizinusöl, ethoxyliert mit 45 mol Ethylenoxid |
| 3,0000 g | Vinylpyrrolidon-Vinylacetat-Copolymer |
| 50,0000 g | Ethanol |
| 0,2000 g | Parfüm |
| 0,0700 g | 1-Amino-4-(2',3'-dihydroxypropyl)-amino-5-chlor-2-nitrobenzol |
| 0,0500 g | Basic Brown 17 (C.1. 12 251) |
| 0,0023 g | Basic Violett 14 (C.1. 42 510) |
| 0,0100 g | Basic Blue 7 (C.1. 42 595) |
| 45,8177 g | Wasser |
| 100,0000 g | |

| Beispiel 10: Schaumfestiger-Konzentrat | |
|---|---|
| 5,0 g | Hanföl |
| 10,0 g | hydriertes Rizinusöl, ethoxyliert mit 45 mol Ethylenoxid |
| 20,0 g | Polyvinylpyrrolidon |
| 10,0 g | Dimethylsiloxan-glykol Copolymer (Belsil DMC 6031 der Firma Wacker/Deutschland) |
| 55,0 g | Wasser |
| 100,0 g | |

| Beispiel 11: Schaumfestiger mit starker Festigung | |
|---|---|
| 0,25 g | Krambeöl |
| 0,20 g | 2-Pyrrolidon-5-carbonsäure |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |

(fortgesetzt)

| Beispiel 11: Schaumfestiger mit starker Festigung | |
|---|---|
| 96,70 g | Wasser |
| 100,00 g | |

| Beispiel 12: Flüssigfestiger mit starker Festigung | |
|---|---|
| 1,00 g | Leindotteröl |
| 1,00 g | Vnylacetat-Crotonsäure-Copolymer, 60%-ige Lösung in Isopropanol/Wasser (ARISTOFLEX® der Firma Hoechst/Deutschland) |
| 2,58 g | Glycerin |
| 50,00 g | Ethanol |
| 0,50 g | Parfümöl |
| 44,92 g | Wasser |
| 100,00 g | |

| Beispiel 13: Gelförmiger Festiger mit starker Festigung | |
|---|---|
| 0,15 g | Krambeöl |
| 0,10 g | 2-Pyrrolidon-5-carbonsäure |
| 3,00 g | Vinylpyrrolidon-Vinylacetat-Copolymer |
| 1,00 g | Hydroxyethylcellulose |
| 5,00 g | Glycerin |
| 90,75 g | Wasser |
| 100,00 g | |

| Beispiel 14: Schaumfestiger für strapaziertes Haar | |
|---|---|
| 0,25 g | Leindotteröl |
| 2,00 g | Polyvinylpyrrolidon |
| 0,30 g | $\alpha$-Hydro-$\omega$-hydroxy-polyoxydimethylsilylen, 13%ig in Cyclodimethylpolysiloxan (Dow Corning Q2 1401 der Firma Dow Coming Europe/Belgien) |
| 5,00 g | 1,2-Propylenglykol |
| 0,10 g | Cetyltrimethylammoniumchlorid |
| 0,10 g | Isopropanol |
| 92,25 g | Wasser |
| 100,00 g | |

| Beispiel 15: Schaumfestiger für extra starken Halt | |
|---|---|
| 0,15 g | Krambeöl |
| 0,10 g | 2-Pyrrolidon-5-carbonsäure |
| 2,00 g | Polyvinylpyrrolidon |
| 2,00 g | Glukosesirup, 64% Oligosaccharide (C-PUR® 01924 der Firma Cerestar/Belgien) |
| 0,30 g | Polyoxyethylen (4) laurylether |
| 0,10 g | Cetyltrimethylammoniumchlorid |
| 0,10 g | Isopropanol |
| 40,00 g | Ethanol |
| 55,25 g | Wasser |
| 100,00 g | |

| Beispiel 16: Gelförmiger Festiger mit natürlichen Polymeren | |
|---|---|
| 0,15 g | Leindotteröl |
| 5,00 g | Glukosesirup, 64% Oligosaccharide (C-PUR® 01924 der Firma Cerestar/Belgien) |
| 5,00 g | Sorbitsirup |
| 2,00 g | Mutterkomharz (Sclerotium Gum) |
| 87,85 g | Wasser |
| 100,00 g | |

| Beispiel 17: Sprühfestiger zum Fönen | |
|---|---|
| 0,15 g | Krambeöl |
| 0,75 g | Vinylpyrrolidon-Vinylacetat-Copolymer |
| 30,00 g | Ethanol |
| 3,00 g | Propylenglykol |
| 0,75 g | Isopropanol |
| 65,35 g | Wasser |
| 100,00 g | |

| Beispiel 18: Non-Aerosol Haarspray | |
|---|---|
| 0,25 g | Krambeöl |
| 3,00 g | Vinylpyrrolidon-Vinylacetat-Copolymer |
| 96,75 g | Ethanol |
| 100,00 g | |

| Beispiel 19: Farbfestiger | |
|---|---|
| 0,2500 g | Krambeöl |
| 3,0000 g | Vinylpyrrolidon-Vinylacetat-Copolymer |
| 50,0000 g | Ethanol |
| 0,2000 g | Parfüm |
| 0,0700 g | 1-Amino-4-(2',3'-dihydroxypropyl)-amino-5-chlor-2-nitrobenzol |
| 0,0500 g | Basic Brown 17 (C.1. 12 251) |
| 0,0023 g | Basic Violett 14 (C.1. 42 510) |
| 0,0100 g | Basic Blue 7 (C.1. 42 595) |
| 46,4177 g | Wasser |
| 100,0000 g | |

| Beispiel 20: Schaumfestiger-Konzentrat | |
|---|---|
| 5,00 g | Leindotteröl |
| 20,00 g | Polyvinylpyrrolidon |
| 10,00 g | Dimethylsiloxan-glykol Copolymer (Belsil DMC 6031 der Firma Wacker/Deutschland |
| 65,00 g | Wasser |
| 100,00 g | |

| Beispiel 21: Schaumfestiger mit starker Festigung | |
|---|---|
| 0,25 g | Zedemholzöl |
| 0,20 g | 2-Pyrrolidon-5-carbonsäure |
| 2,00 g | Polyviriylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |
| 96,70 g | Wasser |
| 100,00 g | |

| Beispiel 22: Flüssigfestiger mit starker Festigung | |
|---|---|
| 1,00 g | Zedernholzöl |
| 1,00 g | Vinylacetat-Crotonsäure-Copolymer, 60%-ige Lösung in Isopropanol/Wasser (ARISTOFLEX® der Firma Hoechst/Deutschland) |
| 2,58 g | Glycerin |
| 50,00 g | Ethanol |
| 0,50 g | Parfümöl |
| 44,92 g | Wasser |
| 100,00 g | |

| Beispiel 23: Gelförmiger Festiger mit starker Festigung | |
|---|---|
| 0,15 g | Zedemholzöl |
| 0,10 g | 2-Pyrrolidon-5-carbonsäure |
| 3,00 g | Vinylpyrrolidon-Vinylacetat-Copolymer |
| 1,00 g | Hydroxyethylcellulose |
| 5,00 g | Glycerin |
| 90,75 g | Wasser |
| 100,00 g | |

| Beispiel 24: Schaumfestiger für strapaziertes Haar | |
|---|---|
| 0,15 g | Zedemholzöl |
| 2,00 g | Polyvinylpyrrolidon |
| 0,30 g | 13% a-Hydro-w-hydroxy-polyoxydimethyl-silylen in Cyclodimethylpolysiloxan (Dow Coming Q2 1401 der Firma Dow Coming Europe/Belgien) |
| 5,00 g | 1,2-Propylenglykol |
| 0,10 g | Cetyltrimethylammoniumchlorid |
| 0,10 g | Isopropanol |
| 92,35 g | Wasser |
| 100,00 g | |

| Beispiel 25: Schaumfestiger für extra starken Halt | |
|---|---|
| 0,15 g | Zedemholzöl |
| 0,10 g | 2-Pyrrolidon-5-carbonsäure |
| 2,00 g | Polyvinylpyrrolidon |
| 2,00 g | Glukosesirup, 64% Oligosaccharide (C-PUR® 01924 der Firma Cerestar/Belgien) |

(fortgesetzt)

| Beispiel 25: Schaumfestiger für extra starken Halt | |
|---|---|
| 0,30 g | Polyoxyethylen (4) laurylether |
| 0,10 g | Cetyltrimethylammoniumchlorid |
| 0,10 g | Isopropanol |
| 40,00 g | Ethanol |
| 55,25 g | Wasser |
| 100,00 g | |

| Beispiel 26: Gelförmiger Festiger mit natürlichen Polymeren | |
|---|---|
| 0,15 g | Zedemholzöl |
| 5,00 g | Glukosesirup, 64% Oligosaccharide (C-PUR® 01924 der Firma Cerestar/Belgien) |
| 5,00 g | Sorbitsirup |
| 2,00 g | Mutterkomharz (Sclerotium Gum) |
| 87,85 g | Wasser |
| 100,00 g | |

| Beispiel 27: Sprühfestiger zum Fönen | |
|---|---|
| 0,10 g | Zedemholzöl |
| 0,75 g | Vinylpyrrolidon-Vinylacetat-Copolymer |
| 30,00 g | Ethanol |
| 3,00 g | Propylenglykol |
| 0,75 g | Isopropanol |
| 65,40 g | Wasser |
| 100,00 g | |

| Beispiel 28: Non-Aerosol Haarspray | |
|---|---|
| 0,15 g | Zedemholzöl |
| 0,10 g | 2-Pyrrolidon-5-carbonsäure |
| 3,00 g | Vinylpyrrolidon-Vinylacetat-Copolymer |
| 50,00 g | Ethanol |
| 46,75 g | Wasser |
| 100,00 g | |

| Beispiel 29: Farbfestiger | |
|---|---|
| 0,2500 g | Zedemholzöl |
| 3,0000 g | Vinylpyrrolidon-Vinylacetat-Copolymer |
| 50,0000 g | Ethanol |
| 0,2000 g | Parfüm |
| 0,0700 g | 1-Amino-4-(2',3'-dihydroxypropyl)-amino-5-chlor-2-nitrobenzol |
| 0,0500 g | Basic Brown 17 (C.1. 12 251) |
| 0,0023 g | Basic Violett 14 (C.1. 42 510) |
| 0,0100 g | Basic Blue 7 (C.1. 42 595) |
| 46,4177 g | Wasser |
| 100,0000 g | |

| Beispiel 30: Schaumfestiger-Konzentrat | |
|---|---|
| 5,0 g | Zedemholzöl |
| 20,0 g | Polyvinylpyrrolidon |
| 10,0 g | Dimethylsiloxan-glykol Copolymer (Belsil DMC 6031 der Firma Wacker/Deutschland |
| 65,0 g | Wasser |
| 100,00 g | |

| Beispiel 31: Schaumfestiger mit den Ölen kombiniert | |
|---|---|
| 0,05 g | Zedemholzöl |
| 0,05 g | Leindotteröl |
| 0,05 g | Krambeöl |
| 0,05 g | Hanföl |
| 2,00 g | Polyvinylpyrrolidon |
| 0,30 g | 13% a-Hydro-w-hydroxy-polyoxydimethyl-silylen in Cyclodimethylpolysiloxan (Dow Coming Q2 1401 der Firma Dow Coming Europe/Belgien) |
| 5,00 g | 1,2-Propylenglykol |
| 0,10 g | Cetyltrimethylammoniumchlorid |
| 0,10 g | Isopropanol |
| 92,30 g | Wasser |
| 100,00 g | |

**Patentansprüche**

1. Haarbehandlungsmittel mit einem Gehalt an

   (A) mindestens einem natürlichen Öl ausgewählt aus Zedemholzöl, Krambeöl, Leindotteröl und Hanföl und

   (B) mindestens einem filmbildenden, haarfestigenden Polymer.

2. Mittel nach Anspruch 1, **dadurch gekennzeichnet, daß** es 0,01 bis 10 Gewichtsprozent des natürlichen Öls enthält.

3. Mittel nach einem der vorstehenden Ansprüche, **dadurch gekennzeichnet, daß** es 0,01 bis 50 Gewichtsprozent der Komponente (B) enthält.

4. Mittel nach einem der vorstehenden Ansprüche, **dadurch gekennzeichnet, daß** es ein organisches oder ein wasserhaltiges organisches Lösungsmittel enthält.

5. Mittel nach einem der vorstehenden Ansprüche, **dadurch gekennzeichnet, daß** es zusätzlich entweder ein Treibmittel enthält oder mit einer mechanischen Sprühvorrichtung betrieben wird.


**Claims**

1. Hair-treatment composition with a content of

   (A) at least one natural oil chosen from cedarwood oil, crambe oil, dodder oil and hemp oil and

   (B) at least one film-forming hair-setting polymer.

2. Composition according to Claim 1, **characterized in that** it comprises 0.01 to 10 per cent by weight of the natural oil.

3. Composition according to one of the preceding claims, **characterized in that** it comprises 0.01 to 50 per cent by weight of component (B).

4. Composition according to one of the preceding claims, **characterized in that** it comprises an organic or a water-containing organic solvent.

5. Composition according to one of the preceding claims, **characterized in that** it additionally comprises either a propellant or is operated with a mechanical spray device.

**Revendications**

1. Composition de traitement capillaire comprenant

   (A) au moins une huile naturelle choisie parmi l'huile de bois de cèdre, l'huile de crambe, l'huile de caméline et l'huile de chanvre, et

   (B) au moins un polymère filmogène fixateur des cheveux.

2. Composition selon la revendication 1, **caractérisée en ce qu'**elle comprend de 0,01 à 10 pour cent en poids de l'huile naturelle.

3. Composition selon l'une quelconque des revendications précédentes, **caractérisée en ce qu'**elle comprend de 0,01 à 50 pour cent en poids du composant (B).

4. Composition selon l'une quelconque des revendications précédentes, **caractérisée en ce qu'**elle comprend un solvant organique ou un solvant organique contenant de l'eau.

5. Composition selon l'une quelconque des revendications précédentes, **caractérisée en ce que** soit elle comprend en outre un agent propulseur soit elle est opérée avec un dispositif de pulvérisation mécanique.